# EUROPEAN PATENT SPECIFICATION

(11) **EP 1 671 653 B1**
(45) Date of publication and mention of the grant of the patent: **01.08.2007**
(21) Application number: 05025659.3
(22) Date of filing: 24.11.2005
(51) Int. Cl.: A61K 47/48, A61K 31/385

(54) **Process for preparing an alpha lipoic acid/cyclodextrin complex and product prepared**
Verfahren zur Herstellung eines alpha Liponsäure Cycldextrin Komplexes sowie derartig hergestellte Produkte
Procédé de préparation d'un complexe d'acide alpha lipoique et cyclodextrine ainsi que produits obtenus

(30) Priority: 17.12.2004 EP 04400070
(43) Date of publication of application: 21.06.2006
(73) Proprietor: Wacker Chemie AG, 81737 München (DE)
(72) Inventor: Reuscher, Helmut, Dr., Onsted, Michigan 49265 (US); Bauer, Mark, Adrian, Michigan 49221 (US)
(74) Representative: Potten, Holger

(56) References cited:
- EP-A- 0 654 484
- EP-A- 1 514 877
- WO-A-00/64440
- TONG LIN-HIU, PANG ZHENG-ZHI, YI YING: "Inclusion Complexes of alpha and beta Cyclodextrin with alpha Lipoic Acid" JOURNAL OF INCLUSION PHENOMENA AND MOLECULAR RECOGNITION IN CHEMISTRY, vol. 23, 1995, pages 119-126, XP009046559
- TRENTIN MARCO ET AL: "Capillary zone electrophoresis study of cyclodextrin: Lipoic acid host-guest interaction." ELECTROPHORESIS, vol. 23, no. 24, December 2002 (2002-12), pages 4117-4122, XP002325022 ISSN: 0173-0835

## Description

The invention relates to a process for preparing an alpha-lipoic acid/cyclodextrin complex and product prepared.

Cyclodextrins are cyclic oligosaccharides composed of 6, 7 or 8 α(1-4)-linked anhydroglucose units. The α-, β- or y-cyclodextrins, which are prepared for example by enzymatic conversion of starch, differ in the diameter of their hydrophobic cavity and are generally suitable for inclusion of numerous lipophilic substances.

Alpha-lipoic acid (Thioctic Acid) is a naturally occurring molecule produced by both plants and animals. It serves as a cofactor for some enzymes and as an excellent antioxidant/free radical scavenger. Since it has been known that alpha-Lipoic Acid can improve insulin sensitivity in cases of Type II diabetes, alpha-Lipoic Acid has been employed for treating diabetes as well as alcoholic liver disease and other neuropathies.

Alpha lipoic acid is a lipid-soluble substance. It is a yellowish powder and is unstable in light. Its solubility in water is very poor. A formulation with cyclodextrins, especially with alpha cyclodextrin is stable to temperature and light and increases the dispersibility in water and the bioavailability. The complexes also reduces unpleasant odour of alpha lipoic acid.

The complexation of lipoic acid with alpha and beta cyclodextrin using a solution method is described in the Journal of Inclusion Phenomena and Molecular Recognition in Chemistry, (1995), vol. 23, pp. 119-96. The processes described therein for preparing a cyclodextrin complex with alpha lipoic acid show various disadvantages in the laboratory and production scale. On use of the solution method on the production scale, the amount of water needed to solubilize the cyclodextrin is enormous. For economic reasons, therefore, these methods cannot be used to prepare a cyclodextrin / alpha lipoic acid complex on the production scale. According to this literature the thermal stability of alpha lipoic acid can be improved significantly by its inclusion in beta cyclodextrin. Calculation of the stability constants for the alpha lipoic acid/cyclodextrin complex according to the Benesi-Hildebrand procedure are described also on page 121 of this paper. The so calculated stability constants for the cyclodextrin complex with alpha cyclodextrin were 3.34 and for the beta cyclodextrin complex 3.95. According to these findings the beta cyclodextrin complex is more stable than the alpha cyclodextrin alpha lipoic acid complex.

In the Canadian patent application 2,135,535 a process is described characterized in that thioctic acid is suspended in water, cyclodextrins are added at elevated temperature such as 50 °C, stirred for several hours, cooled down and the inclusion compound is isolated by filtration and vacuum dried.

Working in suspension to produce cyclodextrins complexes requires elevated temperatures and long complexation times for efficient results. Elevated temperatures can have a negative impact on the stability of the alpha lipoic acid.

It is an object of the present invention to provide a process which allows to prepare a cyclodextrin / alpha lipoic acid complex in a rapid and uncomplicated manner even at room temperature without the disadvantages of the prior art.

This is achieved by a two step process which comprises as a first step dissolving an alpha lipoic acid and a cyclodextrin in an aqueous alkaline solution having a pH above 7, and as a second step adding an acid to lower the pH of the solution to a pH below pH 7.

By this process a mixture of solids in water comprising the cyclodextrin/alpha lipoic acid complex is obtained without high temperatures and long mixing times. The costs of the production process are reduced significantly.

The cyclodextrins used are alpha, beta, or gamma cyclodextrin, preferably beta or alpha cyclodextrin, most preferable alpha cyclodextrin.

The Alpha lipoic acid used is either racemic or enatiomeric pure.

The solvent used is preferably water / alcohol mixtures especially preferred water.

Preferably the process starts from an aqueous alpha lipoic acid solution above pH 7 to which the cyclodextrin is added. However, it is equally possible to prepare an aqueous cyclodextrin solution above pH 7 first and to add Lipoic Acid thereto.

The pH of the aqueous solution during the first step is preferably above between pH 10 and pH 14, especially preferred above between pH 13 and pH 14 or at pH 14.

In case of the precense e.g. of amorphous silica as flowing agent the solution will still be slightly cloudy.

The pH above pH 7 is preferably adjusted by using a suitable base, especially preferred by a base selected from the group ammonium hydroxide, sodium- and potassium hydroxide.

The solubilization of the alpha lipoic acid and the cyclodextrin in the first step is effected using a base and standard mixing equipment.

The solids content of the aqueous solution in the first step is preferably less than 50% (w/w). The solids content can be calculated by dividing the sum of the dry weights of the cyclodextrin, alpha lipoic acid, and base by the overall weight of the mixture.

The aqueous mixture in this first step preferably contains a solids content between 10 and 50% by weight, preferably between 20 and 40%, particularly preferred between 25 and 35%.

The molar ratio cyclodextrin to alpha lipoic acid is preferably between 2.0 and 1.0 more preferably between 1.5 to 1.0, particularly preferred between 1.2 and 1.0, and most preferred between 1.15 and 1.0.

The process of the invention takes place in a temperature range of 10-40°C. It is preferably carried out at 15-30°C, especially preferred at about 25°C. The complexation ordinarily takes place under atmospheric pressure.

The reaction (complexation) time of the first step of the process is preferably below 3 h, especially preferred below 2 h, and most preferred below 1 h.

In the second step of the process according to the present invention the formed alpha lipoic acid/cyclodextrin complex is precipitated by addition of an acid.

An acid selected from the group of inorganic or organic acids is used.

Preferably an inorganic or organic acid selected from the group hydrochloric acid, sulphuric acid, phosphoric acid, acetic acid, citric acid, or ascorbic acid is used.

Especially preferred an inorganic acid selected from the group hydrochloric acid, sulphuric acid, or phosphoric acid is used.

This step turns the lipoic acid back to its acid form, precipitates the complex and brings the pH of the final aqueous mixture below pH 7.

The final pH of the aqueous mixture in this second step is preferably between pH 6 and pH 3, especially preferred between pH 5 and pH 3.

The complex can be used directly in the form of the reaction mixture, however, they may also be isolated e.g. by filtration, centrifugation, belt drying, or spray-drying methods, and processed to give a stable powder.

The complex is preferably dried by oven drying, spray drying, filtration or belt drying, most preferably by spray-drying.

The final reaction mixture containing the alpha lipoic acid/cyclodextrin complex is preferably spray dried in a spray dryer having an inlet temperature higher than 160 °C and an outlet temperature below 100 °C yielding a fine yellow-white powder. The inlet spray drying temperature preferably is higher than 170 °C with an outlet temperature below 90°C, especially preferably higher than 180 °C with an outlet temperature below 80°C.

The alpha lipoic acid/cyclodextrin complexes produced according to this invention show a higher degree of complexation and provide better temperature stability in powder form.

Therefore the invention also relates to an alpha lipoic acid/cyclodextrin complex produced according to the process of the present invention.

An alpha lipoic acid complex produced according to the present invention contains less than 10% of non complexed material, preferably less than 8%, especially less than 5% measured using Differential Scanning Calorimeter (see e.g. example 7).

An alpha lipoic acid/cyclodextrin complex produced according to the Canadian patent application 2,135,535 shows about 28% of not complexed alpha lipoic acid (see comparison example and example 7). In comparison the method described in the present application produces an alpha lipoic acid/cyclodextrin complex with significantly lower percent of non complexed alpha lipoic acid, even up to 100% complexation rate (see example 7).

The superiority of complexes produced according to the method described in this patents leads to significantly higher temperature stability (see example 8, 9, and 10).

According to published heating experiments at 100 °C the half life of lipoic acid at 100 °C is 5.2 h. For the alpha lipoic acid/beta cyclodextrin complex it is 73 h (see Journal of Inclusion Phenomena and Molecular Recognition in Chemistry, (1995), vol. 23, page 122 and figure 4). From these measurements it can be calculated the beta CD complex is about 14 times more stable at 100 °C (73h/5.2h) than the alpha lipoic acid itself. The alpha cyclodextrin/alpha lipoic acid complex according to the present invention shows a half life of more than 213 days and is thus at least 70 times more stable than the lipoic acid itself (see example 10).

The complex according to the present invention may be used in pharmaceutical formulations, dietary supplements, and nutraceutical, or functional food formulations.

The following examples are intended to illustrate the invention in greater detail, but should not be construed as limiting the scope of the invention in any way.

Alpha, beta, and gamma cyclodextrin can be purchased under the name Cavamax from Wacker Chemie, Munich, Germany.

Alpha-lipoic acid can be purchased from Degussa, Freising, Germany. The lipoic acid may also contain a flowing agent such as amorphous silica. Comparison example: (According to example 7 of the Canadian Patent Application 2,135,535)

172.8 g alpha cyclodextrin containing approx. 10% water were dissolved in 700 g water at 50°C. 3.3 g Sodium ascorbate was added and dissolved to the alpha cyclodextrin solution. 33.01 g sieved thioctic acid was added to the alpha cyclodextrin solution and stirred 3 hours at 50°C. The suspension was then filtered and the filtrate was cooled (50 °C, 12 h). The precipitate hereby formed was separated by filtration and dried in a vaccuum oven at 30°C. This product contained 15.8% alpha lipoic acid by weight according to HPLC analysis (see example 6) and was used for comparison purposes in examples 7 and 10.

### Example 1: Preparation of a cyclodextrin alpha lipoic acid alpha cyclodextrin complex

117.2 g of a 45% potassium hydroxide solution (0.940 mol) was added to 5.0 kg water and mixed at room temperature until clear.
194.0 g alpha lipoic acid (99%, 0.931 moles) was added to the potassium hydroxide solution and mixed until clear.
1,046.0 g alpha cyclodextrin (9.25% water content, 0.977 moles) was added and stirred 3 hours until slightly cloudy to clear (in case of the precense amourhous silica as flowing agent the solution will be sligtly cloudy).
0.940 mol of 12 N hydrochloric acid was slowly added until the pH reached pH 3 to precipitate the complex.
The final aqueous mixture is spray dried immediately at 180°C.

### Example 2: Preparation of an alpha lipoic acid/alpha cyclodextrin complex

186.7 g of a 45% potassium hydroxide solution (1.498 moles) was added to 3910 g water and mixed at room temperature until clear.
300 g alpha lipoic acid (99%, 1.439 moles) was added to the potassium hydroxide solution and mixed until clear clear (in case of the precense amourhous silica as flowing agent the solution will be slightly cloudy).
1,572 g alpha cyclodextrin (at 9.25% water 1.468 moles) was added and stirred for 3 hours at room temperature until no further clarification was seen.
1.498 moles of 37% hydrochloric acid was added to precipitate the complex. With a minimal molar excess of hydrochloric acid in comparison to the potassium hydroxide used the final pH of the solution was at pH 3. The final aqueous mixture was spray dried immediately at 180°C.

### Example 3: Preparation of an alpha lipoic acid/alpha cyclodextrin complex

1.44 moles of potassium hydroxide were added to 5.5 kg of water and mixed at room temperature until clear. The pH of this solution was at pH 14.
300 g alpha lipoic acid (98%, 1.425 moles) was added to the potassium hydroxide solution and mixed until clear (in case of the precense amourhous silica as flowing agent the solution will be slightly cloudy).
1.553 kg of dry alpha cyclodextrin (1.598 moles) was added and stirred 50 minutes at room temperature.
1.44 moles of 37% hydrochloric acid was added precipitating the complex. This mixture was stirred 30 minutes at room temperature. The final pH was at about pH 3.
This aqueous mixture was spray dried immediately at about 180°C inlet and about 80°C outlet temperature.

### Example 4: Preparation of an alpha lipoic acid/alpha cyclodextrin complex

23.9 g of a 45% potassium hydroxide solution (0.192 moles) was added to 767 g water and mixed at room temperature until clear.
40.0 g alpha lipoic acid (98%, 0.190 moles) was added to the potassium hydroxide solution and mixed at room temperature until clear (in case of the precense amourhous silica as flowing agent the solution will be slightly cloudy).
221.7 g alpha cyclodextrin (9.2% water content, 0.207 moles) was added and stirred for 60 minutes at room temperature. 0.192 moles of 37% hydrochloric acid was added to precipitate the complex. With a minimal molar excess of hydrochloric acid in comparison to the potassium hydroxide used the final pH of the solution was about pH 3. The final aqueous mixture is spray dried immediately at 180°C.

### Example 5: Preparation of an alpha lipoic acid/alpha cyclodextrin complex

29.95 kg of potassium hydroxide pellets (87.9%, 469.3 moles) were added to 1920.0 kg of water and mixed at room temperature until clear. The pH of this solution was at pH 14.
97.8 kg alpha lipoic acid (98%, 464.5 moles) was added to the potassium hydroxide solution and mixed until clear (in case of the precense amourhous silica as flowing agent the solution will be slightly cloudy).
555.1 kg alpha cyclodextrin (8.9% water content, 520.3 moles) was added and stirred 60 minutes at room temperature.
469.3 moles of 37.7% hydrochloric acid was added precipitating the complex. This mixture was stirred 55 minutes at room temperature. The final pH was at about pH 3.
The final aqueous mixture was spray dried immediately at about 180°C inlet and about 80°C outlet temperature.

### Example 6: High-Performance Liquid Chromatography analysis

The alpha lipoic acid/cyclodextrin complex was analyzed for content using High-Performance Liquid Chromatography (HPLC). The column used is a 25mm C18.

### Conditions:

UV detector at wavelength 215 nm, flowrate 0.8 ml/min. Mobile phase is comprised of 585 ml ethanol, 90 ml Acetonitrile, 455 ml 0.05 mol potassium di hydrogen phosphate (KH₂PO₄), adjusted to pH 3.0-3.1 with phosphoric acid (H₃PO₄). The injection volume was 20 micro liters (µl) and the column temperature 30°C. Under these conditions the retention time for alpha lipoic acid is approximately 6.5 minutes.

### Procedure:

150 mg of cyclodextrin complex was sonicated for 60 seconds in 50 ml acetonitrile. 40 ml water was added and sonicated for another 30 seconds. The solution was transferred to a 100 ml volumetric flask and filled to the mark with water. This solution was then tested by HPLC and the alpha lipoic acid content content calculated using a multipoint calibration curve prepared from a known standard.

### Results:

| HPLC Samples | Alpha lipoic acid content (w/w) [%] |
|---|---|
| Comparison Example | 15.8 |
| Example 1 | 13.4 |
| Example 2 | 13.6 |
| Example 3 | 12.5 |
| Example 4 | 13.5 |
| Example 5 | 12.7 |

### Example 7: Differential Scanning Calorimetry analysis for uncomplexed alpha lipoic acid.

Cyclodextrin complexes (3-6 mg sample size) were analyzed using Differential Scanning Calorimeter (DSC). Samples were scanned for an alpha lipoic acid (ALA) melting peak around 60°C. This peak, if present, was compared to a peak at this temperature of pure alpha lipoic acid to calculate percentage of uncomplexed alpha lipoic acid in the complex. Taking the sample size and the alpha lipoic acid content of the sample into account from the area under the curve (AUC) the Joule per g melting energy for uncomplexed alpha lipoic acid can be calculated. The peak around 60°C decreases with increasing dregree of complexation. A cyclodextrin complex containing no uncomplexed alpha lipoic acid shows no melting peak anymore around 60°C.

### Results:

| | Content ALA 1%] | Sample Weight [mg] | AUC at 60°C [mJ] | Peak at 60°C [J/g] | Non complexed ALA [%] | Complexed ALA [%] |
|---|---|---|---|---|---|---|
| Pure ALA | 98.0 | 3.057 | 377.40 | 126 | 100 | 0.0 |
| Comp. Ex. | 15.8 | 4.890 | 27.526 | 36 | 28.3 | 11.3 |
| Ex. 3 | 12.5 | 2.890 | 0 | 0 | 0 | 12.5 |
| Ex. 4 | 13.5 | 4.040 | 0 | 0 | 0 | 13.5 |
| Ex. 5 | 12.7 | 5.662 | 2.673 | 4 | 3.2 | 12.3 |

Even with a lower alpha lipoic acid content in the complex, the amount of complexed alpha lipoic acid is significatly higher in product produced according to the present application (12.3% to 13.5% versus 11.3% in the comparison example).

### Example 8: Temperature stability at 25°C

Alpha lipic acid/cyclodextrin complexes were stored in a closed polypropylene container at 25°C. Samples were taken from time to time and the alpha lipoic acid content was measured via HPLC according to example 6. The initial alpha lipoic acid content was set to 100%.

### Results:

| Days of storage | Content ALA [%] Pure ALA | Content ALA [%] Example 5 |
|---|---|---|
| 0 | 100 | 100 |
| 5 | | 100 |
| 14 | | 100 |
| 39 | 100 | |
| 45 | | 97 |
| 67 | 100 | |
| 76 | | 97 |
| 107 | 100 | 97 |
| 135 | | 97 |

The pure alpha lipoic acid is stable at room temperature. The alpha lipoic acid in the cyclodextrin complex according to this invention is also stable at room temperature after some small initial losses, which correspond to the uncomplexed alpha lipoic acid in the complex (the complex in example 5 has 3% uncomplexed alpha lipoic acid according to DSC measurements in example 7).

### Example 9: Temperature stability at 40°C

Alpha lipoic acid/cyclodextrin complexes were stored in a closed polypropylene container in a drying oven at 40°C. Samples were taken from time to time and the alpha lipoic acid content was measured via HPLC according to example 6. The initial alpha lipoic acid content was set to 100%.

### Results:

| Days of storage | Content ALA [%] Pure ALA | Content ALA [%] Example 5 |
|---|---|---|
| 0 | 100 | 100 |
| 5 | | 97 |
| 8 | 99 | |
| 14 | | 96 |
| 28 | | 94 |
| 39 | 48*) | |
| 45 | | 93 |
| 59 | | 87 |
| 76 | | 86 |
| 81 | 41*) | |
| 107 | | 86 |
| 135 | | 86 |

| | | |
|---|---|---|
| *) difficult to obtain a homogeneous sample, because product polymerizes | | |

The alpha lipoic acid in the cyclodextrin complex according to this invention shows a significantly higher stability than the pure alpha lipoic acid and stays stable at 40 °C after some initial losses.

### Example 10: Temperature stability at 100 °C

Alpha lipoic acid/cyclodextrin complexes were stored in closed glass containers in a drying oven at 100°C. Samples were taken from time to time and the alpha lipoic acid content was measured via HPLC according to example 6. The initial alpha lipoic acid content was set to 100%.

### Results:

| Days of storage | Content ALA [%] Pure ALA | Content ALA [%] Comparison Example | Content ALA [%] Example 3 |
|---|---|---|---|
| 0 | 100 | 100 | 100 |
| 3 | 38 | 85 | |
| 6 | | | 96 |
| 9 | | | 93 |
| 11 | 13 | | |
| 13 | | | 91 |
| 14 | | 77 | |
| 24 | | 72 | |
| 28 | 4 | | |
| 36 | 0 | | |
| 53 | | | 80 |
| 55 | | 35 | |
| 84 | | | 77 |
| 112 | | | 72 |
| 125 | | 10 | |
| 143 | | | 66 |
| 173 | | | 60 |
| 213 | | | 54 |

The half life of pure alpha lipoic acid under the described storage condition at 100 °C is less than three days. The half life of alpha lipoic acid complex (example 3) is more than 213 days. This means the complex produced according to this invention under these conditions is more than 70 times more stable than the pure alpha lipoic acid itself (213d/3d). Even after 213 days of storage at 100°C still 54% of the original alpha lipoic acid content is not degraded, whereas only 10% can be detected in product produced according the Canadian patent 2,135,535 just after 125 days.

## Claims

1. Process for the preparation of a cyclodextrin/alpha lipoic acid complex **characterized in that** in a first step of a two step process an alpha lipoic acid and a cyclodextrin are dissolved in an aqueous alkaline solution having a pH above pH 7, and in a second step an acid is added to lower the pH of the solution to a pH below pH 7.

2. Process according to claim 1 **characterized in that** the cyclodextrin is a beta or an alpha cyclodextrin

3. Process according to claim 2, **characterized in that** the cyclodextrin is an alpha cyclodextrin.

4. Process according to one of claims 1 to 3, **characterized in that** the pH of the aqueous solution during the first step is between pH 10 and pH 14.

5. Process according to one of claims 1 to 4, **characterized in that** the pH of the aqueous alkaline solution is adjusted by a base selected from the group ammonium hydroxide, sodium- and potassium hydroxide.

6. Process according to one of claims 1 to 5, **characterized in that** the aqueous mixture in first step contains a solids content between 10 and 50% by weight.

7. Process according to one of claims 1 to 6, **characterized in that** the molar ratio cyclodextrin to alpha lipoic acid is between 2.0 and 1.0.

8. Process according to one of claims 1 to 7, **characterized in that** the reaction time of the first step is below 3 h.

9. Process according to one of claims 1 to 8, **characterized in that** the acid added in the second step is selected from the group acetic acid, citric acid, ascorbic acid, hydrochloric acid, sulphuric acid, or phosphoric acid.

10. Process according to one of claims 1 to 9, **characterized in that** the molar ratio cyclodextrin to alpha lipoic acid is between 2.0 and 1.0.

11. Process according to one of claims 1 to 10, **characterized in that** the final pH of the aqueous solution in this second step is between pH 6 and pH 3.

12. Process according to one of claims 1 to 11, **characterized in that** the complex is used directly in the form of the reaction mixture or is isolated by filtration, centrifugation, belt drying, or spray-drying methods.

13. Process according to claim 12, **characterized in that** the reaction mixture is spray dried in a spray dryer having an inlet temperature higher than 160 °C and an outlet temperature below 100 °C.

14. Alpha lipoic acid/cyclodextrin complex produced according to one of claims 1 to 13.

15. Alpha lipoic acid/cyclodextrin complex according to claim 14 having a half life of more than 213 days at 100 °C.

16. Alpha lipoic acid/cyclodextrin complex according to claim 13 or 14 containing less than 10% of non complexed alpha lipoic acid.

17. Use of a complex according to one of claims 14 to 16 in pharmaceutical formulations, dietary supplements, and nutraceutical, or functional food formulations.

## Patentansprüche

1. Verfahren zur Herstellung eines Cyclodextrin/alpha-Liponsäure-Komplexes, **dadurch gekennzeichnet, daß** man in einem ersten Schritt eines zweischrittigen Verfahrens eine alpha-Liponsäure und ein Cyclodextrin in einer wäßrig-alkalischen Lösung mit einem pH-Wert über 7 löst und in einem zweiten Schritt zur Senkung des pH-Werts der Lösung auf einen pH-Wert unter pH 7 eine Säure zugibt.

2. Verfahren nach Anspruch 1, **dadurch gekennzeichnet, daß** es sich bei dem Cyclodextrin um ein beta- oder alpha-Cyclodextrin handelt.

3. Verfahren nach Anspruch 2, **dadurch gekennzeichnet, daß** es sich bei dem Cyclodextrin um ein alpha-Cyclodextrin handelt.

4. Verfahren nach einem der Ansprüche 1 bis 3, **dadurch gekennzeichnet, daß** der pH-Wert der wäßrigen Lösung während des ersten Schritts zwischen pH 10 und pH 14 liegt.

5. Verfahren nach einem der Ansprüche 1 bis 4, **dadurch gekennzeichnet, daß** man den pH-Wert der wäßrig-alkalischen Lösung mit einer Base aus der Gruppe Ammoniumhydroxid, Natriumhydroxid und Kaliumhydroxid einstellt.

6. Verfahren nach einem der Ansprüche 1 bis 5, **dadurch gekennzeichnet, daß** die wäßrige Mischung im ersten Schritt einen Feststoffgehalt zwischen 10 und 50 Gew.-% aufweist.

7. Verfahren nach einem der Ansprüche 1 bis 6, **dadurch gekennzeichnet, daß** das Molverhältnis von Cyclodextrin zu alpha-Liponsäure zwischen 2,0 und 1,0 liegt.

8. Verfahren nach einem der Ansprüche 1 bis 7, **dadurch gekennzeichnet, daß** die Reaktionszeit des ersten Schritts unter 3 h liegt.

9. Verfahren nach einem der Ansprüche 1 bis 8, **dadurch gekennzeichnet, daß** man die im zweiten Schritt zugegebene Säure aus der Gruppe Essigsäure, Citronensäure, Ascorbinsäure, Salzsäure, Schwefelsäure oder Phosphorsäure auswählt.

10. Verfahren nach einem der Ansprüche 1 bis 9, **dadurch gekennzeichnet, daß** das Molverhältnis von Cyclodextrin zu alpha-Liponsäure zwischen 2,0 und 1,0 liegt.

11. Verfahren nach einem der Ansprüche 1 bis 10, **dadurch gekennzeichnet, daß** der End-pH-Wert der wäßrigen Lösung im zweiten Schritt zwischen pH 6 und pH 3 liegt.

12. Verfahren nach einem der Ansprüche 1 bis 11, **dadurch gekennzeichnet, daß** man den Komplex direkt in Form der Reaktionsmischung verwendet oder durch Filtrations-, Zentrifugations-, Bandtrocknungs- oder Sprühtrocknungsmethoden isoliert.

13. Verfahren nach Anspruch 12, **dadurch gekennzeichnet, daß** man die Reaktionsmischung in einem Sprühtrockner mit einer Einlaßtemperatur über 160°C und einer Auslaßtemperatur unter 100°C sprühtrocknet.

14. alpha-Liponsäure/Cyclodextrin-Komplex, hergestellt nach einem der Ansprüche 1 bis 13.

15. alpha-Liponsäure/Cyclodextrin-Komplex nach Anspruch 14 mit einer Halbwertszeit von mehr als 213 Tagen bei 100°C.

16. alpha-Liponsäure/Cyclodextrin-Komplex nach Anspruch 13 oder 14 mit weniger als 10% nichtkomplexierter alpha-Liponsäure.

17. Verwendung eines Komplexes nach einem der Ansprüche 14 bis 16 in pharmazeutischen Formulierungen, Nahrungsergänzungsmitteln und Nutraceutical- oder Functional-Food-Formulierungen.

## Revendications

1. Procédé de préparation d'un complexe de cyclodextrine et d'acide alpha lipoïque **caractérisé en ce que**, dans une première étape d'un procédé en deux étapes, un acide alpha lipoïque et une cyclodextrine sont dissous dans une solution alcaline aqueuse ayant un pH supérieur à pH 7 et que, dans une seconde étape, un acide est ajouté pour abaisser le pH de la solution à un pH inférieur à pH 7.

2. Procédé selon la revendication 1, **caractérisé en ce que** la cyclodextrine est une bêta ou une alpha cyclodextrine.

3. Procédé selon la revendication 2, **caractérisé en ce que** la cyclodextrine est une alpha cyclodextrine.

4. Procédé selon l'une quelconque des revendications 1 à 3, **caractérisé en ce que** le pH de la solution aqueuse durant la première étape est entre pH 10 et pH 14.

5. Procédé selon l'une quelconque des revendications 1 à 4, **caractérisé en ce que** le pH de la solution alcaline aqueuse est ajusté par une base choisie parmi le groupe constitué de l'hydroxyde d'ammonium, de l'hydroxyde de sodium et de potassium.

6. Procédé selon l'une quelconque des revendications 1 à 5, **caractérisé en ce que** le mélange aqueux dans la première étape a une teneur en solides entre 10 et 50% en poids.

7. Procédé selon l'une quelconque des revendications 1 à 6, **caractérisé en ce que** le rapport molaire de la cyclodextrine à l'acide alpha lipoïque est entre 2,0 et 1,0.

8. Procédé selon l'une quelconque des revendications 1 à 7, **caractérisé en ce que** le temps de réaction de la première étape est inférieur à 3 heures.

9. Procédé selon l'une quelconque des revendications 1 à 8, **caractérisé en ce que** l'acide ajouté dans la seconde étape est choisi parmi le groupe constitué de l'acide acétique, de l'acide citrique, de l'acide ascorbique, de l'acide chlorhydrique, de l'acide sulfurique ou de l'acide phosphorique.

10. Procédé selon l'une quelconque des revendications 1 à 9, **caractérisé en ce que** le rapport molaire de la cyclodextrine à l'acide alpha lipoïque est entre 2,0 et 1,0.

11. Procédé selon l'une quelconque des revendications 1 à 10, **caractérisé en ce que** le pH final de la solution aqueuse dans cette seconde étape est entre pH 6 et pH 3.

12. Procédé selon l'une quelconque des revendications 1 à 11, **caractérisé en ce que** le complexe est utilisé directement sous la forme du mélange réactionnel ou est isolé par des procédés de filtration, centrifugation, séchage sur bande ou séchage par pulvérisation.

13. Procédé selon la revendication 12, **caractérisé en ce que** le mélange réactionnel est séché par pulvérisation dans un séchoir atomiseur ayant une température d'entrée supérieure à 160°C et une température de sortie inférieure à 100°C.

14. Complexe d'acide alpha lipoïque et de cyclodextrine produit selon l'une quelconque des revendications 1 à 13.

15. Complexe d'acide alpha lipoïque et de cyclodextrine selon la revendication 14 ayant une demi-vie de plus de 213 jours à 100°C.

16. Complexe d'acide alpha lipoïque et de cyclodextrine selon la revendication 13 ou 14 contenant moins de 10% d'acide alpha lipoïque non complexé.

17. Utilisation d'un complexe selon l'une quelconque des revendications 14 à 16 dans des formulations pharmaceutiques, des suppléments diététiques et des formulations de nutraceutiques ou d'aliments fonctionnels.
